# EUROPEAN PATENT SPECIFICATION

(11) **EP 0 621 350 B1**
(45) Date of publication and mention of the grant of the patent: **05.03.1997**
(21) Application number: 94105972.7
(22) Date of filing: 18.04.1994
(51) Int. Cl.: C23C 14/06, C23C 14/16, C23C 28/02, A61B 17/06

(54) **Metallic article possessing a brightly colored surface of low reflectivity and process for producing such surface**
Metallischer Formkörper, der eine hellfarbige Oberfläche mit niedrigem Reflektionsvermögen besitzt, sowie Verfahren zur Herstellung einer solchen Oberfläche
Article métallique avec surface de couleur claire peu réfléchissante et procédé pour la production d'une telle surface

(30) Priority: 23.04.1993 US 52119
(43) Date of publication of application: 26.10.1994
(73) Proprietor: United States Surgical Corporation, Norwalk, Connecticut 06856 (US)
(72) Inventor: Lauro, Michael P., Lincoln, RI 02865 (US); Avissar, Jacob, Providence, RI 02906 (US); Granger, Richard N., Huntington, CT 06484 (US); Muth, Ross R., Brookfield, CT 06804 (US); Proto, George R., West Haven, CT 06516 (US)
(74) Representative: Marsh, Roy David

(56) References cited:
- EP-A- 0 494 648
- DE-C- 3 841 443
- FR-A- 2 592 063
- GB-A- 2 162 864
- PATENT ABSTRACTS OF JAPAN vol. 017, no. 236 (C-1057) 13 May 1993 & JP-A-04 365 849 (TOYODA GOSEI CO LTD) 17 December 1992

## Description

### BACKGROUND OF THE INVENTION

This invention relates to a metallic article whose surface appearance has been altered by the application of a series of metal-containing coatings thereto. More particularly, the present invention relates to a metallic article, e.g., a stainless steel surgical needle, possessing a multilayer coating exhibiting a brightly colored but relatively nonreflective appearance for increased visual contrast of the article against its background, and to a physical vapor deposition (PVD) process for producing the coating.

Surgical needles possessing dark nonreflective surfaces which increase their visibility are known. U.S. Patent Nos. 4,905,695, 4,959,068 and 4,968,362 describe such needles and chemical methods by which the dark nonreflective surfaces can be produced.

Physical vapor deposition (PVD) embodies a number of related techniques, e.g., cathode sputtering, D.C. sputtering, ion plating and arc evaporation deposition, which have been used to apply a variety of metal-containing coatings to a metal workpiece so as to improve or modify one or more characteristics of its surface such as its corrosion resistance, hardness, color, etc.

German Patent No. 3,841,443 describes a surgical needle whose surfaces are coated with a wear resistant metal coating employing a PVD technique. The surgical needle to be coated is connected to a negative electrical potential of up to 600 V. The metal to be applied to the needle is evaporated at a cathode under a vacuum of from 10⁻³ to 10⁻¹ mbar in the presence of a suitable gas to form a nitride, carbide or oxide of the metal on the surface of the needle, e.g., titanium nitride or titanium carbide.

### SUMMARY OF THE INVENTION

In accordance with the present invention, a metallic article, e.g., a stainless steel surgical needle, is provided having a multilayer coating possessing a brightly colored surface of low reflectivity, the coating comprising:
a) a layer of elemental metal;
b) a layer of brightly colored metal carbon nitride of high reflectivity overlaying the layer of elemental metal; and,
c) a reflectivity-diminishing layer of metal carbide overlaying the second layer of brightly colored metal carbon nitride.

Further in accordance with the present invention, a process is provided for applying a brightly colored coating of low reflectivity to a metallic article which comprises:
a) coating the surface of a metallic article with a layer of elemental metal, the formation of the elemental metal layer being effected under physical vapor deposition conditions in the absence of chemically reactive gas;
b) coating the layer of elemental metal with a layer of brightly colored metal carbon nitride of high reflectivity, the formation of the latter layer being effected under physical vapor deposition conditions in the presence of a chemically reactive gas; and,
c) coating the layer of brightly colored metal carbon nitride of high reflectivity with a layer of reflectivity-diminishing metal carbide the formation of the latter layer being effected in the presence of chemically reactive gas, the thickness of the layer of reflectivity-diminishing metal carbide being such as to appreciably diminish the reflectivity of the layer of brightly colored metal carbon nitride without significantly diminishing its bright color.

The expressions "bright color" and "brightly colored" are used herein to designate hues such as golden yellow, bronze, etc., which provide a relatively high level of contrast against a background of low or moderate color intensity. The expressions "low reflectivity" and "nonreflective" are used herein to designate the light reflective characteristics of a treated metallic surface compared with those of the untreated metallic surface, the latter generally exhibiting a relatively high level of reflectivity.

### BRIEF DESCRIPTION OF THE DRAWINGS

Fig. 1 schematically illustrates a cathode sputtering apparatus which can be used in accordance with the process of this invention to provide the surface of a stainless steel surgical needle with a bright colored, relatively nonreflective multilayer coating;
Fig. 1A illustrates a portion of the surgical needle mounting fixture employed in the apparatus of Fig. 1; Fig. 2 schematically illustrates in plan view the cathode sputtering apparatus of Fig. 1; and,
Fig. 3 schematically illustrates in plan view a cathode sputtering apparatus similar to that of Fig. 2 but possessing two coating zones for the deposition of two different metals and/or metal-containing compounds.

### DESCRIPTION OF THE PREFERRED EMBODIMENTS

While the process of this invention can be carried out upon any metallic article, it is particularly well suited to treating a surgical needle, e.g., of surgical grade stainless steel such as the 300 or 400 series stainless steels, preferably the latter.

Other suitable metals for the fabrication of surgical needles which can be utilized herein include the quaternary alloys disclosed in U.S. Patent Nos. 3,767,385 and 3,816,920, the contents of which are incorporated by reference herein. A suitable quaternary alloy possesses the following ranges of components:

| Component | Broad Range | Preferred Range | Most Preferred Range |
|---|---|---|---|
| Nickel | 10-50 | 24-45 | 30-40 |
| Cobalt | 10-50 | 25-45 | 30-40 |
| Nickel + Cobalt | 50-85 | 60-80 | 65-75 |
| Chromium | 10-30 | 12-24 | 15-22 |
| Molybdenum, tungsten and/or niobium (columbium) | 5-20 | 8-16 | 10-13 |

A particular quaternary alloy which is suitable for the manufacture of a surgical needle which can be coated in accordance with of this invention, designated MP35N, is available in wire form from Maryland Specialty Wire, Inc., Cockeysville, Maryland and contains (nominal analysis by weight) : nickel, 35%; cobalt, 35%; chromium, 20% and molybdenum, 10%.

The metal or metals which are selected for application to the surface of the metallic article can be selected from amongst any of the metals whose carbon nitride provides a bright color and whose carbide when superimposed on the carbon nitride diminishes the latter's reflectivity without significantly diminishing its bright color. Suitable metals include Al, V, Cr, Fe, Co, Ni, Cu, Zn, Ge, Y, Mo, Ru, Rh, Pd, Ag, Au, Cd, In, Sn, Sb, Ti, Ta, W, Ir, Nb and Pt. For surgical needles, Ti has been found to provide especially good results.

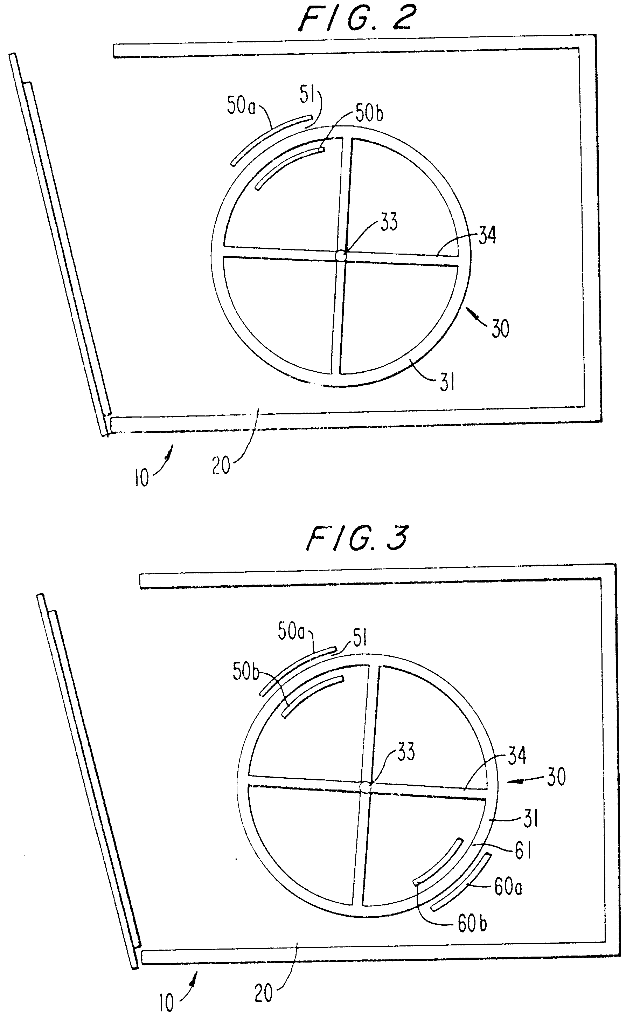

While it is often a matter of convenience to employ a single metal, it is also within the scope of the invention to use a different metal or combination of metals in a given coating step to achieve differing effects. Thus, as shown in Fig. 3, cathode metal plates 50a and 50b of a first metal, e.g., titanium, occupy a first coating zone 51 while cathode metal plates 60a and 60b of a second metal, e.g., aluminum, gold, niobium, tungsten, zirconium, etc., occupy a second coating zone 61. Many other arrangements and combinations are, of course, possible.

The metal is applied to the surface of the metallic object under PVD conditions in a sequence of coating steps, each step resulting in the deposition of a layer of specific composition. Thus, the initial coating step is carried out in the absence of a chemically reactive atmosphere and results in the deposition of the metal in its elemental form upon the surface of the needle. In general, the conditions of this coating step can be such as to lay down a metal layer having a thickness of from about 5 to about 200 x 10⁻⁸m (about 0.05 to about 2 microns), and preferably from about 10 to about 100 x 10⁻⁸m (about 0.1 to about 1 microns). The subsequent coating step is carried out in the presence of a gaseous mixture of nitrogen and a hydrocarbon, e.g, ethylene, and results in the deposition upon the previously deposited elemental metal layer of a layer of metal carbon nitride having a thickness of from about 10 to about 500 x 10⁻⁸m (about 0.1 to about 5 microns), and preferably from about 1 to about 4 x 10⁻⁶m (about 1 to about 4 microns). This metal carbon nitride layer possesses a desirably bright color, e.g., golden yellow, bronze, or the like, which provides good visual contrast against most background hues the needle is likely to encounter in use. However, the bright color also exhibits a relatively high level of reflectivity which, in the case of a surgical needle, can offset the benefit of greater visual contrast. Accordingly, in the next coating step, the atmosphere of the PVD environment is changed to that of a hydrocarbon gas, e.g., ethylene, resulting in deposition of a layer of metal carbide which has the effect of reducing the reflectivity of the underlying metal carbon nitride layer without, however, appreciably diminishing the bright color of the latter. In general, the metal carbide layer can be deposited to a thickness of from about 5 to about 200 x 10⁻⁸ m (about 0.5 to about 2 microns) and preferably from about 10 to about 100 x 10⁻⁸m (about 0.1 to about 1 microns). If necessary or desirable, an additional coating step can be carried out under the same or similar conditions as the second coating step to deposit another layer of brightly colored metal carbon nitride. This further coating step would ordinarily be carried out only if the previous coating step were to result in an excessive diminution in the bright coloration of the previously deposited metal carbon nitride layer.

A preferred PVD technique employs the well known procedure of cathode sputtering which involves placing the coating metal and the metallic article to be coated in a heated vacuum chamber, e.g., maintained under a pressure of from about 133 to about 0.133 x mPa (about 10⁻³ to about 10⁻⁶ Torr) and a temperature of from about 80°C to about 180°C the coating metal constituting the cathode and the metallic article and its support means constituting the anode. The chamber is provided with electrical connectors and controls, sources of nitrogen gas and hydrocarbon gas a heating means, e.g., a resistance coil, and means for rotating the support means for the metallic article(s) within the chamber to the disclosure of U.S. Patent No. 4,895,765.

It is further within the scope of this invention to siliconize a surgical needle which has been coated in accordance with the present invention, preferably employing the siliconization method disclosed in US-A-5258013, the contents of which are incorporated by reference herein.

The amount of force required to pass a surgical needle through tissue, i.e., the penetration force, has been found to be significantly less for a surgical needle which has been coated and subsequently siliconized in accordance with this invention than a surgical needle which has merely been coated or siliconized. Briefly described, the preferred siliconization procedure comprises applying to a surface of the previously coated needle a siliconization material comprising an aminoalkyl siloxane and at least one other silicone copolymerizable therewith and thereafter curing the siliconization material to provide an adherent silicone coating on the needle. One suitable method for achieving siliconization of the needle utilizes the siliconization material and procedures described in U.S. Patent No. 3,574,673.

The siliconization material includes (a) from about 5-20 weight percent of an aminoalkyl siloxane of the formula 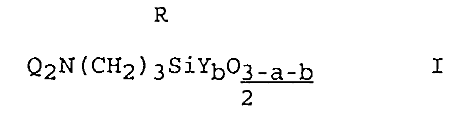 in which R is a lower alkyl radical containing no more than about 6 carbon atoms; Y is selected from the group consisting of _OH and _OR' radicals in which R' is an alkyl radical of no more than 3 carbon atoms; Q is selected from the group consisting of hydrogen, _^{C}H₃ and _^{C}H₂CH₂NH₂; a has a value of 0 or 1, and b has a value of 0 or 1 and the sum of a+b has a value of 0, 1 or 2 , and (b) from about 80 to 95 weight percent of a methyl substituted siloxane of the formula 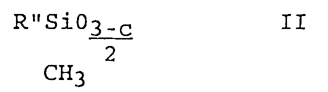 in which R" is selected from the group consisting of _OH and _CH₃ radicals and c has a value of 1 or 2. In addition to, or in lieu of, the foregoing second copolymerizable siloxane, one can use one or more cyclosiloxanes, e.g., as described in the "Encyclopedia of Polymer Science and Engineering", Mark et al., eds., 2nd ed., John Wiley & Son (1989), Vol. 15, p. 207 et seq., the contents of which are incorporated by reference herein, provided, of course, the total amount of second copolymerizable siloxane(s) is within the aforestated range.

A particularly preferred siliconization material is Do Corning Corporation's Dow Corning® MDX 4-4159 Fluid ("MDX Fluid"), a 50 percent active solution of dimethyl cyclosiloxanes and dimethoxysilyldimethylamino-ethylaminopropyl silicone polymer in a mixture of Stoddard solvent (mineral spirits) and isopropyl alcohol.

MDX Fluid or other siliconization fluid can be applied to a surface of the cleaned surgical needle by dipping, wiping, spraying, etc., in the form of a dilute organic solution, e.g., prepared with a solvent such as hexane, trichlorotrifluoroethane, 1,1,1-trichloroethane or mineral spirits. In general, it is preferred to dilute the siliconization material in a hydrocarbon solvent possessing from 5 to 10 carbon atoms, e.g., pentane, hexane (which is preferred), heptane, octane, etc. MDX Fluid cures at room temperature to provide an adherent silicone coating.

Spraying is a preferred method of applying the siliconization fluid, at least in the case of a surgical needle possessing a suture-receiving axial bore, or recess, or a surgical possessing a reduced shank end. In the case of the latter, it is preferred to insert the shank portion of the needle into a support block, e.g., of rigid foam, and thereafter to spray the siliconization fluid onto the exposed surface of the needle. Since the shank end of the needle is embedded in the support block, it will remain free of silicone during the spraying procedure. The use of a support block can, of course, also be employed in the case of the axial recess type needle to prevent siliconization material from entering the recess. It is preferable that the coated needle while still in its support block be subjected to curing conditions. If this involves heat, it will, of course, be necessary to select a support block material which can withstand the elevated temperature selected for curing.

The process of the invention will now be illustrated with the coating of a curved stainless steel surgical needle employing titanium as the coating metal.

### EXAMPLE 1

A quantity of stainless steel surgical needles of known type, each having an axial bore at its blunt end for receiving the tip of a suture at the time of suture-needle attachment, are cleaned by submersion in three consecutive baths of 99 weight percent ethanol or other suitable solvent. The effectiveness of the solvent cleaning operation can be enhanced, if desired, by ultrasonic cleaning the details of which are well known in the art. The solvent-wetted needles from the third bath are placed in a clean, dry ceramic dish where they are allowed to dry, advantageously by exposure to hot air, e.g., at a temperature of from 25 to 95°C and preferably at 80°C.

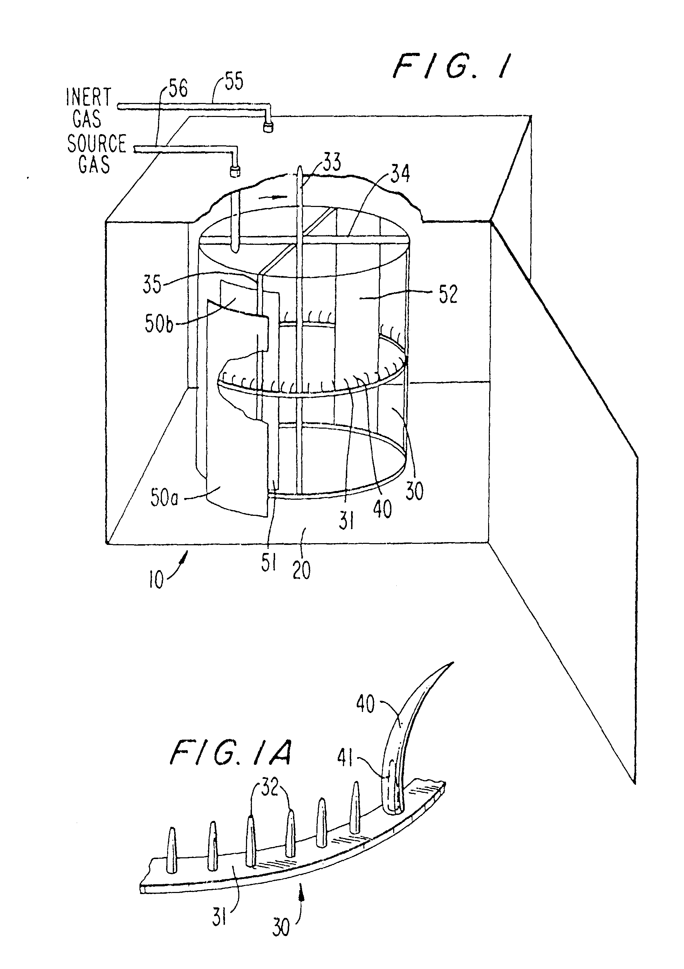

As shown in Fig. 1, the cleaned needles are placed on a drum-like rotatable needle mounting fixture 30 which is positioned within heated vacuum chamber 20 of cathode sputtering unit 10 which is generally of a known type. As shown in the enlarged section of mounting fixture 30 illustrated in Fig. 1A, the fixture includes a circular rack 31 possessing a number of pins 32. Each pin is intended to be received within axial bore 41 (shown in dotted outline) formed in the blunt end of a surgical needle 40 thus providing a means for mounting a quantity of needles in chamber 20 and moving the needles in a circular path within the chamber. While Fig. 1 shows but a single needle mounting fixture 30 in place, in practice, a number of such fixtures, all supported on rotatable shaft 33 through crossarm supports 34 and vertical members 35, would be installed in vacuum chamber 20. The radius from the center of shaft 33 to the edge of rack 31 can vary considerably and is advantageously from about 10 to about 20cm.

As shown in Figs. 1, 2 and 3, needle mounting fixture 30 with needles 40 supported thereon is connected as the anode and titanium (99% pure) plates, or "targets", 50a and 50b are connected as the cathode to a current source by suitable electric connectors and controls. Space 51 lying between the titanium plates is referred to as the "sweet zone". A shutter plate 52 which may be made of the same material as the workpiece, e.g., stainless steel in the case of a surgical needle, possesses such a configuration as to occupy the entirety of sweet zone 51 when interposed between titanium plates 50a and 50b.

Following sealing of vacuum chamber 20, a vacuum of about 10mPa (7.5 x 10⁻⁵ Torr) is drawn on the chamber. The chamber is heated to 120°C for 5 minutes with the needle mounting fixture rotating at about 1 rpm with a flow of argon of 400 SCCM (standard cubic centimers per minute).

An optional plasma etch cleaning step is advantageously carried out to slowly remove oxides from the surface of the needles prior to the commencement of the coating process herein. This optional cleaning step can be carried out in stages, e.g., in three stages. In the first stage, the needle mounting fixture is rotated in the vacuum chamber for 5 revolutions at about 0.8 rpm with a voltage and current of 490-500V and 0.08-0.1A, respectively. Concurrently, targets 50a and 50b carry a charge of 270-300V and a current of 0.6-0.12A with a gas flow of 300 SCCM argon. In the second stage, the needle mounting fixture is rotated for 4 revolutions at about 0.8 rpm with a voltage and current of 690-700V and about 0.1A, respectively, the targets carrying a charge of 270-300V and a current of 0.06-0.12A with a gas flow of 300 SCCM argon. In the third stage of the optional plasma cleaning step, the needle mounting fixture is rotated for 3 revolutions at about 0.8 rpm with a voltage and current of 890-900V and about 0.1A, respectively, the targets carrying a charge of 270-300V and a current of 0.06-0.12A with a gas flow of 420 SCCM argon.

As a result of the plasma etch cleaning operation, oxides removed from the anode, i.e., needle mounting fixture 30 and needles 40, will deposit upon the cathode, i.e., titanium plates 50a and 50b, from which they must be removed prior to the start of the coating process for otherwise they will redeposit upon the anode surfaces. To remove the oxides from the titanium plates, mounting fixture 30 is maintained in a nonrotating state with shutter plate 52 disposed between the titanium plates. A voltage of about 270-300V and a current of about .06-0.12A applied over a period of about 2 to 3 minutes causes the oxides, probably with some titanium metal, to be removed from the titanium plates and deposited upon the shutter plate.

To coat the plasma etched surface of the needles, pure titanium is dynamically applied to the needles to a thickness of about 1.8 - 2.2 x 10⁻⁷m (0.18-0-22 microns) employing a charge of 475-495V and a current of 7-7.2A in the presence of a gas flow of 200 SCCM argon and employing a negative bias of 135V on the needle substrate.

In the next coating operation, a layer of titanium carbon nitride is dynamically applied to the previously applied titanium layer to a thickness of about 2.4 - 3.0 x 10⁻⁷m (0.24-0.3 microns) employing a charge of 548-558V and a current of 7.0-7.2A in the presence of a mixture of argon at 198 SCCM, nitrogen at 150 SCCM and ethylene at 11 SCCM and employing a negative bias of 120V on the needle substrate. The resulting titanium carbon nitride exhibits a bright bronze color. To diminish the reflectivity of the titanium carbon nitride layer, a layer of titanium carbide is dynamically applied thereto to a thickness of about 2.4 to 2.6 x 10⁻⁷m (0.24-0.26 microns) employing a charge of 520-540V and a current of 6.0-6.2A in the presence of a mixture of argon at 198 SCCM and ethylene at 50 SCCM and employing a negative bias of 120V on the needle substrate. The resulting titanium carbide layer exhibits a grayish hue which diminishes the reflectivity of the underlying titanium carbon nitride layer.

With two minutes of static deposition onto shutter plate 52, targets 50a and 50b are cleaned in preparation for an optional color adjustment step which provides the final color of the needles. In this optional step, a layer of titanium carbon nitride is dynamically applied to the previously applied titanium carbide layer to a thickness of 6.8 - 7.2 x 10⁻⁷m (0.68 - 0.72 microns) employing a charge of 548-558 V and a current of 7.0-7.2A in the presence of a mixture of argon at 198 SCCM, nitrogen at 150 SCCM and ethylene at 11 SCCM and employing a negative bias of 120V on the needle substrate. This final layer exhibited a bronze color of relatively low reflectivity.

The vacuum chamber is cooled over a 5 minute period with a flow of argon of 400 SCCM.

It is also contemplated that numerous color variations can be achieved employing the process of this invention, e.g., by applying different metals in one or more coating steps as previously indicated. In order to facilitate such processes, the vacuum chamber of the cathode sputtering apparatus preferably includes a second coating zone 61 as shown in Fig. 3. The first coating zone 51 is substantially as described above in Figs. 1 and 2. The second coating zone of Fig. 3 preferably possesses targets of a metal from the same family of the periodic table as the metal cathode plates of the first coating zone. For example, when the metal plates of zone 51 are selected to be titanium, the target metal of second zone 61 can advantageously be aluminum, gold, niobium, tungsten or zirconium. During the first coating step, a titanium base layer will be applied substantially as described above while second zone 61 is inactive. During the second coating step, both coating zones would be active so that titanium would be applied in first zcne 51 and the second metal would be applied in the second zone 61. Of course, the first and second coating zones can be activated sequentially with or without a period of simultaneous operation to provide particular results. As in the embodiment of the process described above, gases such as nitrogen and acetylene can be added in the vicinity of the target plates of either or both zones in order to provide a coating of a desired color. The third, brightness reducing layer would then be applied, optionally followed by a comparatively thin fourth color layer.

### EXAMPLE 2

This example illustrates the siliconization process of aforementioned U.S. patent 5258013 to apply a silicone coating to needles which have been coated in accordance with the present invention.

The needles are placed in a basket and immersed in an ultrasonic cleansing unit containing a 2% by weight distilled water solution of Liquinox (an aqueous soap concentrate from Alconox, Inc., New York City) for 5 minutes. The basket is raised to the vapor section of the unit and held there for another 5 minutes. The needles are then dried and after 20 minutes are transferred to a second basket which is immersed for 30 seconds in a siliconization medium prepared from 1 part by volume of MDX Fluid and 9 parts by volume of hexane as solvent. Following drainage of excess siliconization medium, the needles are spread on a tray and heated for 16 hours at 120°C to effect curing of the silicone coating.

### EXAMPLE 3

To compare the tissue penetration characteristics of needles which have been coated in accordance with this invention and those of uncoated needles, and the tissue penetration characteristics of needles which have been coated in accordance with this invention and siliconized in accordance with the process of U.S. patent 5258013 (Example 2, supra) and those of uncoated needles which have been siliconized by the aforesaid method, the penetration forces of 5.5 x 10⁻⁴m (.022 inch) diameter straight taper point surgical needles were measured by individually passing the needles through Porvair (Inmont Corporation), a microporous polyurethane membrane of about 1.06 x 10⁻³m (.042 inches) thickness serving as a simulation of flesh.

Measurement of the needle penetration forces was accomplished using the test procedure and apparatus described in U.S. Patent No. 5,181,416, the contents of which are incorporated by reference herein. The test was performed by a testing fixture and an Instron Universal Testing Machine. The surgical needles were mounted in a gripping clamp which fixed the needle in a position perpendicular to the Porvair surface. The needle was moved into the Porvair which was mounted on top of an Instron load cell. The maximum amount of vertical force is recorded as the needle is pushed through the Porvair.

The needle penetration force measurements were as follows:

| Type of Needle | Average Penetration Force For 10 Needle Samples (Grams) |
|---|---|
| Uncoated, non-siliconized (control) | 420 |
| Uncoated but siliconized | 182 |
| Coated but non-siliconized | 239 |
| Coated and siliconized | 129 |

As these data show, surgical needles which have received both of the present treatments exhibit far less average penetration force than surgical needles which have received only one or the other of these treatments.

## Claims

1. A metallic article having applied thereto a multilayer coating possessing a brightly colored surface of low reflectivity, the coating comprising:
a) a layer of elemental metal;
b) a layer of brightly colored metal carbon nitride of high reflectivity overlaying the layer of elemental metal; and,
c) a reflectivity-diminishing layer of metal carbide overlaying the layer of brightly colored metal carbon nitride.

2. The metallic article of Claim 1 wherein the elemental metal is titanium, the metal carbon nitride is titanium carbon nitride and the metal carbide is titanium carbide.

3. The metallic article of Claim 1 or 2 which is a surgical needle.

4. The metallic article of Claim 1, 2 or 3 which is fabricated from an alloy comprising nickel, cobalt, chromium and at least one metal selected from molybdenum, tungsten and niobium.

5. The metallic article of any one of the preceding claims, further comprising a silicone coating applied to the last layer.

6. The metallic article of Claim 5 wherein the silicone coating is obtained from an aminoalkyl siloxane and at least one other siloxane copolymerizable therewith.

7. A process for applying a brightly colored surface of low reflectivity to a metallic article which comprises:
a) coating the surface of a metallic article with a layer of elemental metal, the formation of the elemental metal layer being effected under physical vapor deposition conditions in the absence of chemically reactive gas;
b) coating the layer of elemental metal with a layer of brightly colored metal carbon nitride of high reflectivity, the formation of the latter being effected under physical vapor deposition conditions in the presence of chemically reactive gas; and,
c) coating the layer of brightly colored metal carbon nitride of high reflectivity with a layer of reflectivity-diminishing metal carbide the formation of the latter layer being effected under physical vapor deposition conditions in the presence of chemically reactive gas, the thickness of the reflectivity-diminishing layer of metal carbide being such as to appreciably diminish the reflectivity of the layer of brightly colored metal carbon nitride without significantly diminishing its bright color.

8. The process of claim 7 wherein the surface of the metallic article is substantially free of oxide prior to coating step (a).

9. The process of claim 7 or 8 wherein coating steps (a), (b) and (c) are each carried out under cathode sputtering conditions.

10. The process of claim 7, 8 or 9 wherein the elemental metal, the metal component of the metal carbon nitride and/or the metal component of the metal carbide is one or more metals selected from Al, V, Cr, Fe, Co, Ni, Cu, Zn, Ge, Y, Mo, Ru, Rh, Pd, Ag, Au, Cd, In, Sn, Sb, Ti, Ta, W, Ir, Nb and Pt.

11. The process of claim 10 wherein the elemental metal is titanium, the metal carbon nitride is titanium carbon nitride and the metal carbide is titanium carbide.

12. The process of any one of claims 7 to 11 and further comprising the step of applying a silicone coating to the last layer.

13. The process of claim 12 wherein the silicone coating is obtained from an aminoalkyl siloxane and at least one other siloxane copolymerizable therewith.

14. The process of any one of claims 7 to 13 including the step of selecting as the metallic article a surgical needle.

## Patentansprüche

1. Metallischer Gegenstand mit einer darauf aufgetragenen mehrlagigen Beschichtung, die eine hellfarbige Oberfläche von geringem Reflexionsvermögen besitzt, wobei die Beschichtung umfaßt:
a) eine Lage aus elementarem Metall;
b) eine Lage aus hell farbigem Metallkohlenstoffnitrid hohen Reflexionsvermögens, die über der Schicht aus elementarem Metall liegt; und
c) eine das Reflexionsvermögen vermindernde Schicht aus Metallkarbid, die über der Schicht aus hellfarbigem Metallkohlenstoffnitrid liegt.

2. Metallischer Gegenstand gemäß Anspruch 1, worin das elementare Metall Titan ist, das Metallkohlenstoffnitrid Titankohlenstoffnitrid und das Metallkarbid Titankarbid ist.

3. Metallischer Gegentand gemäß Anspruch 1 oder 2, der eine Operationsnadel ist.

4. Metallischer Gegenstand gemäß Anspruch 1, 2 oder 3, der aus einer Legierung hergestellt ist, die umfaßt Nickel, Kobalt, Chrom und zumindest ein Metall, das von Molybdän, Wolfram und Niob ausgewählt ist.

5. Metallischer Gegenstand gemäß einem der vorhergehenden Ansprüche, weiter umfassend eine Silikonbeschichtung, die auf die letzte Schicht aufgetragen ist.

6. Metallischer Gegenstand gemäß Anspruch 5, worin die Silikonbeschichtung von einem Aminoalkylsiloxan und zumindest einem anderen Siloxan, das damit copolymerisierbar ist, erhalten wird.

7. Verfahren zum Anbringen einer hellfarbigen Oberfläche mit geringem Reflexionsvermögen auf einen metallischen Gegenstand, das umfaßt:
a) Beschichten der Oberfläche eines metallischen Gegenstandes mit einer Schicht aus elementarem Metall, wobei die Bildung der Schicht aus elementarem Metall unter Bedingungen der physikalischen Gasphasenbeschichtung in der Abwesenheit eines chemisch reaktiven Gases erzielt wird;
b) Beschichten der Schicht aus elementarem Metall mit einer Schicht eines hellfarbigen Metallkohlenstoffnitrids hohen Reflexionsvermögens, wobei die Bildung der letzteren unter Bedingungen der physikalischen Gasphasenabscheidung in Anwesenheit von chemisch reaktivem Gas erzielt wird; und
c) Beschichten der Schicht aus hellfarbigem Metallkohlenstoffnitrid mit hohem Reflexionsvermögen mit einer Schicht eines das Reflexionsvermögen vermindernden Metallkarbids, wobei die Bildung der letzteren Schicht unter Bedingungen der physikalischen Gasphasenabscheidung in Anwesenheit von chemisch reaktivem Gas erzielt wird und die Dicke der das Reflexionsvermögen vermindernden Schicht aus Metallkarbid so ist, daß sie das Reflexionsvermögen der Schicht von hellfarbigem Metallkohlenstoffnitrid merklich verringert, ohne ihre helle Farbe wesentlich abzuschwächen.

8. Verfahren gemäß Anspruch 7, worin die Oberfläche des metallischen Gegenstandes im wesentlichen frei von Oxid vor dem Beschichtungsschritt (a) ist.

9. Verfahren gemäß Anspruch 7 oder 8, worin die Beschichtungsschritte (a), (b) und (c) jeweils unter Kathodenzerstäubungsbedingungen ausgeführt werden.

10. Verfahren gemäß Anspruch 7, 8 oder 9, worin das elementare Metall, die Metallkomponente des Metallkohlenstoffnitrids und/oder die Metallkomponente des Metallkarbids eines oder mehrere Metalle sind, die von Al, V, Cr, Fe, Co, Ni, Cu, Zn, Ge, Y, Mo, Ru, Rh, Pd, Ag, Au, Cd, In, Sn, Sb, Ti, Ta, W, Ir, Nb und Pt ausgewählt sind.

11. Verfahren gemäß Anspruch 10, worin das elementare Metall Titan ist, das Metallkohlenstoffnitrid Titankohlenstoffnitrid und das Metallkarbid Titankarbid ist.

12. Verfahren gemäß einem der Ansprüche 7 bis 11 und weiter umfassend den Schritt des Auftragens einer Silikonbeschichtung auf die letzte Schicht.

13. Verfahren gemäß Anspruch 12, worin die Silikonbeschichtung von einem Aminoalkylsiloxan und zumindest einem anderen Siloxan erhalten wird, das mit diesem copolymerisierbar ist.

14. Verfahren gemäß einem der Ansprüche 7 bis 13, umfassend den Schritt des Auswählens einer Operationsnadel als den metallischen Gegenstand.

## Revendications

1. Article métallique auquel est appliqué un revêtement multicouche, possédant une surface de couleur claire de faible réflectivité, le revêtement comprenant :
a) une couche de métal élémentaire;
b) une couche d'un nitrure de carbone métallique de couleur claire de forte réflectivité recouvrant la couche de métal élémentaire; et
c) une couche diminuant la réflectivité de carbure de métal recouvrant la couche de nitrure de carbone métallique de couleur claire.

2. Article métallique de la revendication 1, où le métal élémentaire est du titane, le nitrure de carbone métallique est du nitrure de carbone et titane et le carbure métallique est le carbure de titane.

3. Article métallique de la revendication 1 ou 2, qui est une aiguille chirurgicale.

4. Article métallique de la revendication 1, 2 ou 3 qui est fabriqué d'un alliage comprenant du nickel, du cobalt, du chrome et au moins un métal sélectionné parmi le molybdène, le tungstène et le niobium.

5. Article métallique selon l'une quelconque des revendications précédentes, comprenant de plus un revêtement de silicone appliqué à la dernière couche.

6. Article métallique de la revendication 5, où le revêtement de silicone est obtenu d'un aminoalkyl siloxane et d'au moins un autre siloxane copolymérisable avec lui.

7. Procédé d'application d'une surface de couleur claire de faible réflectivité à un article métallique qui comprend :
a) le revêtement de la surface de l'article métallique par une couche de métal élémentaire, la formation de la couche de métal élémentaire étant effectuée en conditions de dépôt physique en phase vapeur, en l'absence d'un gaz chimiquement réactif;
b) le revêtement de la couche de métal élémentaire avec une couche d'un nitrure de carbone métallique de couleur claire de forte réflectivité, la formation doe cette dernière étant effectuée en conditions de dépôt physique en phase vapeur, en présence d'un gaz chimiquement réactif; et
c) le revêtement de la couche de nitrure de carbone métallique de couleur claire de forte réflectivité par une couche d'un carbure de métal diminuant la réflectivité, la formation de cette dernière couche étant effectuée en conditions de dépôt physique en phase vapeur, en présence d'un gaz chimiquement réactif, l'épaisseur de la couche diminuant la réflectivité du carbure métallique étant telle que cela diminue, de façon appréciable, la réflectivité de la couche du nitrure de carbone métallique de couleur claire sans diminuer, de manière significative, sa couleur claire.

8. Procédé de la revendication 7, où la surface de l'article métallique est sensiblement sans oxyde avant l'étape de revêtement (a).

9. Procédé de la revendication 7 ou 8, où les étapes de revêtement (a), (b) et (c) sont effectuées en conditions de pulvérisation à la cathode.

10. Procédé de la revendication 7, 8 ou 9, où le métal élémentaire, le composant métallique du nitrure de carbone métallique et/ou le composant métallique du carbure de métal est un ou plusieurs métaux sélectionnés parmi Al, V, Cr, Fe, Co, Ni, Cu, Zn, Ge, Y, Mo, Ru, Rh, Pd, Ag, Au, Cd, In, Sn, Sb, Ti, Ta, W, Ir, Nb et Pt.

11. Procédé de la revendication 10, où le métal élémentaire est le titane, le nitrure de carbone métallique est le nitrure de carbone de titane et le carbure métallique est le carbure de titane.

12. Procédé selon l'une quelconque des revendications 7 à 11, comprenant de plus l'étape d'appliquer un revêtement de silicone à la dernière couche.

13. Procédé de la revendication 12, où le revêtement de silicone est obtenu à partir d'un aminoalkyl siloxane et d'au moins un autre siloxane copolymérisable avec lui.

14. Procédé selon l'une quelconque des revendications 7 à 13, comprenant l'étape de sélection, comme article métallique, d'une aiguille chirurgicale.
